# EUROPEAN PATENT SPECIFICATION

(11) **EP 4 295 756 B1**
(45) Date of publication and mention of the grant of the patent: **22.01.2025**
(21) Application number: 23461523.5
(22) Date of filing: 27.02.2023
(51) Int. Cl.: A61B 5/00, A61B 5/07

(54) **A COMPUTER IMPLEMENTED CLASSIFICATION TOOL AND METHOD FOR CLASSIFICATION OF MICROELECTRODE RECORDINGS TAKEN DURING A DEEP BRAIN STIMULATION**
COMPUTERIMPLEMENTIERTES KLASSIFIZIERUNGSWERKZEUG UND VERFAHREN ZUR KLASSIFIZIERUNG VON WÄHREND EINER TIEFEN HIRNSTIMULATION AUFGENOMMENEN MIKROELEKTRODENAUFZEICHNUNGEN
OUTIL DE CLASSIFICATION MIS EN UVRE PAR ORDINATEUR ET PROCÉDÉ DE CLASSIFICATION D'ENREGISTREMENTS DE MICROÉLECTRODES PRISES PENDANT UNE STIMULATION CÉRÉBRALE PROFONDE

(43) Date of publication of application: 27.12.2023
(60) Divisional application: 24191673.3
(73) Proprietor: Naukowa I Akademicka Siec Komputerowa Panstwowy Instytut Badawczy, 01-045 Warszawa (PL)
(72) Inventor: CIECIERSKI, Konrad, 01-045 Warszawa (PL)
(74) Representative: Markieta, Jaroslaw Franciszek

(56) References cited:
- XIAO LINXIA ET AL: "Amplitude-frequency-aware deep fusion network for optimal contact selection on STN-DBS electrodes", SCIENCE CHINA INFORMATION SCIENCES, SCIENCE CHINA PRESS, BEIJING, vol. 65, no. 4, 10 March 2022 (2022-03-10), XP037730643, ISSN: 1674-733X, [retrieved on 20220310], DOI: 10.1007/S11432-021-3392-1

## Description

The object of this invention is a computer implemented classification tool and method for classification of microelectrode recordings taken during a deep brain stimulation.

During the Deep Brain Stimulation (DBS) surgery for Parkinson's disease (PD), the main goal is to place the permanent stimulating electrode into an area of the brain that becomes pathologically hyperactive. This area, called Subthalamic Nucleus (STN), is small and located deep within the brain. Therefore, the main challenge is precise localisation of the STN region. In this work, there is described a neural network acting as decision support system for the neurosurgical DBS surgery.

Expected target positioning of the stimulating electrode is firstly calculated off-line based on the Computed Tomography (CT) and Magnetic Resonance Imaging (MRI) scans. Unfortunately, based on these data, it is not possible to accurately discriminate the STN area from the surrounding brain tissue. CT and MRI scans give only the approximate location. Precise localization is calculated on-line (during the surgery) based on measurements of the electrical activity of brain tissue recorded by thin microelectrodes.

WO2009002072A2 discloses a method of determining the position of a deep brain stimulation (DBS) electrode which finds the position of the DBS electrode with respect to a deep brain target region, by using a first volume data set containing information on the deep brain target region and a second volume data set containing information on the DBS electrode implanted toward the deep brain target region, and which includes: a first step of generating a subvolume of the deep brain target region from the first volume data set, and also generating a subvolume of the DBS electrode from the second volume data set; and a second step of overlapping and displaying the subvolume of the deep brain target region and the subvolume of the DBS electrode.

WO2020261282A1 discloses a method for determining position of an electrode lead inside a body tissue, including: receiving electrical signals recorded from at least one macro electrode contact of an electrode lead positioned inside a body tissue; extracting spiking (SPK) signals from the received electrical signals; providing stored measurements or indications thereof; determining a position of the lead and/or the at least one macro electrode contact inside said body tissue based on the extracted SPK signals and the provided stored measurements or indications thereof.

EP1396233A1 discloses an apparatus for use in a brain of a subject is provided, including an instrument, adapted to be inserted into the brain. A set of one or more electrodes, coupled to the instrument, are adapted to sense electrical activity of the brain and to transmit an electrical activity signal responsive thereto. A location sensor, adapted to be coupled to the instrument transmits a location signal indicative of a location of the instrument. A control unit, analyzes the electrical activity signal and the location signal. The control unit determines, responsive to the analysis, a position of the instrument with respect to an image of the brain, and electrophysiological information regarding tissue at the position.

The article XIAO LINXIA, ET AL: "Amplitude-frequency-aware deep fusion network for optimal contact selection on STN-DBS electrodes", SCIENCE CHINA INFORMATION SCIENCES, SCIENCE CHINA PRESS, BEIJING, vol. 65 no. 4, 10 March 2022 (2022-03-10), XP037730643, ISSN: 1674-733X, DOI:10.1007/S11432-021-3392-1 discloses a neural network trained on STN signal being a heuristically selected disjoint time frames of signals received from STN-DBS electrodes. Method disclosed indicates the need of filtering input signals as a data preparation step before application of such pre-processed data to the neural; network.

The present invention introduces a method of creating a computer implemented classification tool for classification of microelectrode recordings taken during a deep brain stimulation using deep residual neural network with attention comprising steps collecting a data set of recordings taken during a deep brain stimulation; splitting recordings into overlapping time chunks, and converting time chunks into spectrograms; dividing data set into a training set, a validation set, and a test set. The method according the invention is characterized by the steps of putting each spectrogram into a deep neural network of ResNet architecture augmented with a self-attention layer added after ResNet layers, with a head layer comprising a single 2D convolutional layer followed by batch normalization and ReLU activation function. In this method the network is trained to return zero for time chunks taken from recordings made outside of the STN region of a brain and to return one for time chunks taken form recordings made within the STN region of a brain, fine tuning the network training with the validation set, cross checking the network with the test set.

The method according to the invention is characterized in that the collecting data set includes signal recordings of 10 seconds.

The method according to the invention is characterized in that the recordings are split into overlapping 500 ms long time chunks.

The method according to the invention is characterized in that each spectrogram has dimensions 129 (frequency) by 53 (time).

The method according to the invention is characterized in that the 2D convolutional layer has a kernel size 7x7, padding 3 and 64 filters.

The method according to the invention is characterized in that each ResNet layer consists of six blocks, one lead-in block and five consecutive proper blocks.

The method according to the invention is characterized in that the convolutional layers in ResNet blocks has kernel size 3x3 and padding 1.

The present invention also provides a computer implemented classification tool for classification of microelectrode recordings taken during a deep brain stimulation, comprising an input block for receiving signals from microelectrodes in STN region of a brain; a conversion block for converting signals from the time domain into spectrograms; a computer implemented a deep residual neural network with attention created as in any of claims from 1 to 7 for receiving spectrograms. Further it comprises an output block presenting the result of classification done by the deep residual neural network with attention.

The present invention also introduces a method of classification of microelectrode recordings taken during a deep brain stimulation using deep residual neural network with attention comprising steps of receiving signals from microelectrodes placed in the STN region of a brain in an input block; converting signals from the time domain into spectrograms in a conversion block; providing spectrograms to a computer implemented a deep residual neural network with attention created as in any of claims from 1 to 7 for receiving spectrograms, and presenting the result of classification done by the deep residual neural network with attention in an output block.

Present invention advantageously increase the probability of correct identification of the area within the brain tissue that is suitable for electrostimulation. Signaling from the invention provides additional source of information to the neurosurgeon in respect of the functional placing of electrodes i.e. it helps to identify the best possible place to insert an electrode during Deep Brain Stimulation surgery for Parkinson's disease.

The attentional mechanism occurring after ResNet blocks is crucial in this solution. This mechanism intelligently modifies the output of ResNet blocks so that, in effect, it modifies the error signals or interference so that the quality of the classification that the network performs is the best possible. The modification mechanism can both reduce the amplitude of unwanted interference and amplify the amplitude of the signal in selected areas.

The object of the present invention is described in more details in reference to the preferred embodiments, where:
- Fig. 1: shows an example of a brain scan with electrodes inserted;
- Fig. 2: shows the schematic architecture of the ResNet architecture that has been augmented with a self-attention layer added after each of the ResNet layers according to the preferred embodiment of the invention;
- Fig. 3: shows ResNet blocks;
- Fig. 4: shows a recording with selected chunk containing artifact;
- Fig. 5: shows an amplitude of selected chunk;
- Fig. 6: shows a spectrogram of selected chunk;
- Fig. 7: shows Channel 0;
- Fig. 8: shows Channel 5;
- Fig. 9: shows Channel 12;
- Fig. 10: shows Channel 15;
- Fig. 11: shows a schematic of the attention layer;
- Fig. 12: shows an attention acting on tensor;
- Fig. 13: shows ResNet layer output #1;
- Fig. 14: shows ResNet layer output #2;
- Fig. 15: shows attention vector #1;
- Fig. 16: shows attention vector #2;
- Fig. 17: shows ResNet layer output #2 after attention;
- Fig. 18: shows ResNet layer output #1 after attention;
- Fig. 19: shows ROC curve for chunks;
- Fig. 20: shows ROC curve for recordings;

Expected target positioning of the stimulating electrode is firstly calculated off-line based on the Computed Tomography (CT) and Magnetic Resonance Imaging (MRI) scans. Unfortunately, based on these data, it is not possible to accurately discriminate the STN area from the surrounding brain tissue. CT and MRI scans give only the approximate location. Precise localization is calculated on-line (during the surgery) based on measurements of the electrical activity of brain tissue recorded by thin microelectrodes.

A set of parallel recording microelectrodes is inserted into the brain and advanced in measured steps towards the expected location of the STN. Typically three to five recording microelectrodes are used for each brain hemisphere. The STN area is localized, and the coordinates of the stimulating electrode in the stereotactic surgical space is obtained. Unfortunately, the recorded data are adversely affected by various artifacts that can cause false-positive detection of the STN. Therefore, these artifacts must either be removed in data pre-processing or be properly addressed in the decision process.

By analysing of the recordings, the computer classifier assesses if they were registered within the STN or not. The recordings' classification results provide a 3D localization of the Subthalamic Nucleus (STN) in the stereotactic surgical space. Microelectrode recordings contain various artifacts that can cause false-positive detection of the STN. These artifacts must either be removed in data pre-processing or be properly addressed by the classification algorithm.

Existing solutions are very sensitive to artifacts in the recordings and require complex data pre-processing to remove them. The described neural network-based approach does not require any prior removal of the artifacts.

The implementation of described network provides a novel method for calculating the expected position of the stimulating electrode based on the measurements of electrical activity of brain tissue. The neural network with attention is used during surgery to classify the microelectrode recordings and determine final position of the stimulating electrode within the STN area.

The test were carried on a number of data collected during DBS surgeries, giving encouraging results. The experimental results demonstrate that deep learning methods augmented with self-attention blocks are competing to the other solutions. They provide significant robustness to measurement uncertainty and improve an accuracy of the stimulating electrode placement.

This work describes an approach to classify the recordings using the residual neural network with attention in the temporal domain. The residual neural network has been augmented by including the self-attention blocks between the residual blocks. Obtained results show that attention acting along the temporal domain diminishes the influence of the artifacts. It is also evident that attention has improved the learning process and classification results.

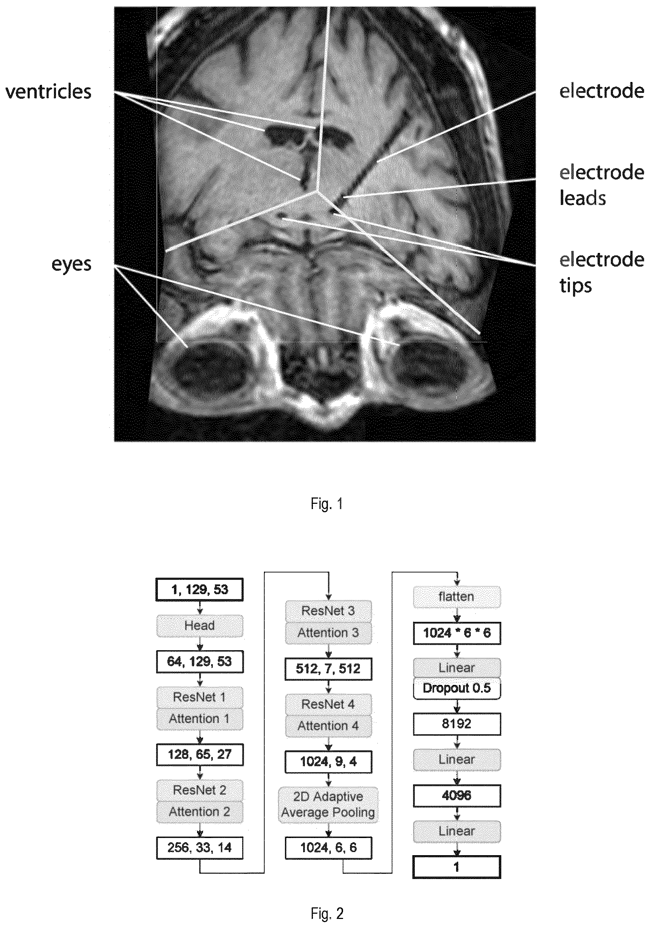

Figure 1 shows an example of a brain scan with electrodes inserted. The leads of the electrodes are located in the STN, dorsal to the axial plane traversing the eyes. Yellow lines separate the cut-out planes used for visualization. It can be readily seen how deeply the STN is located and how difficult and, at the same time, crucial it is to determine its stereotactic position with maximal precision.

In the progression of the PD disease due to degenerative changes in SNr, the STN becomes hyperactive. This change presents itself in recordings as a higher volume of background noise and increased power of the recorded signals in certain frequency bands. Unfortunately, artifacts present in microelectrode recordings also increase such attributes. If not adequately addressed, this, in turn, leads to false-positive errors, i.e., recordings made outside of the STN having wrong labels pointing to the STN, all because of the presence of the artifacts. In the case of a standard approach to this problem artifacts are detected and removed prior calculation of the classification attributes.

In the solution described in this paper, for successful classification, the network architecture addresses the problem of artifacts.

Analysed recording is split into overlapping 500 ms long chunks for classification. These chunks are then converted into spectrograms using the Python package SciPy. Each spectrogram has dimensions 129 (frequency) by 53 (time). Spectrograms are then input to the neural network and are classified individually.

The described network is based upon the ResNet architecture that has been augmented with a self-attention layer added after each of the ResNet layers. Schematic architecture is shown in Figure 2.

Boxes with a white background show the shape of the passing tensor. The shape of the tensor is given in PyTorch notation, i.e., Channels, Height, and Width. For simpler notation, the batch dimension has been omitted on all figures. The network was trained to return zero for chunks taken from recordings made outside of the STN and one for chunks taken from recordings made within the STN.

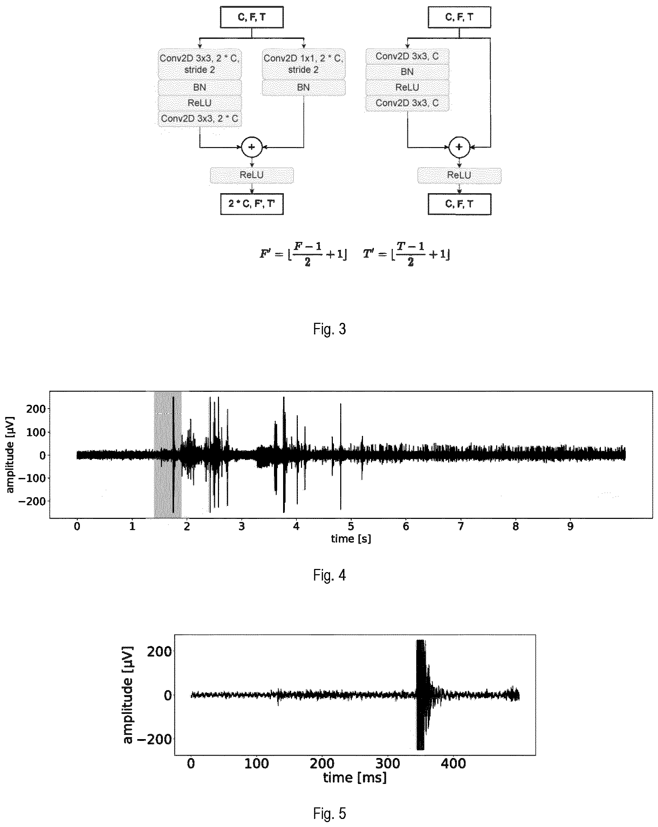

The Head layer comprises a single 2D Convolutional layer followed by Batch Normalization and ReLU activation function. The Convolutional layer has kernel size 7x7, padding 3 and 64 filters. Each Resnet layer consists of six blocks, one lead-in block (shown on the left), and five consecutive proper blocks (shown on the right). All convolutional layers in ResNet blocks use kernel size 3x3 and padding 1. ResNet blocks are shown in Figure 3.

### Attention in temporal domain

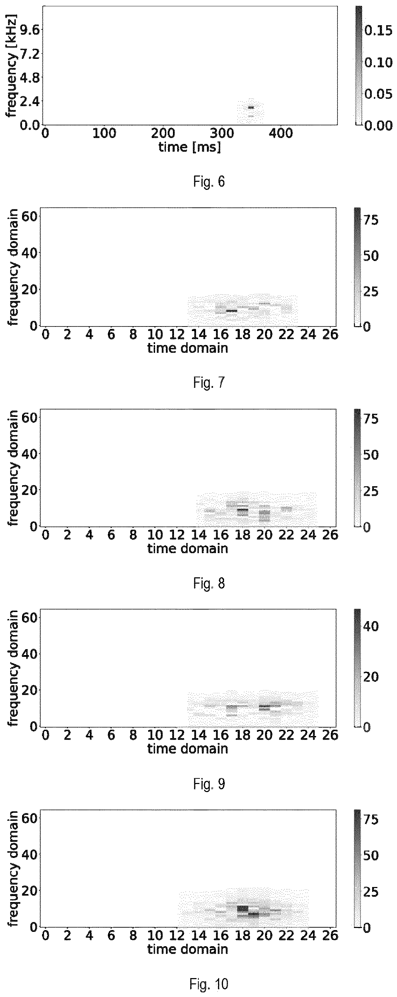

As can be seen in Figure 4 - Figure 6, the artifact present in the amplitude of the recorded signal (Figure 4 and Figure 6) is strongly present not only in the spectrogram spanning selected time period (Figure 6) but also across the channels in the output of the ResNet block (Figure 7 - Figure 10).

As channels are computed using different convolutional filters, in some of the channels the artifact is more pronounced than in others (compare Figure 7 and Figure 9). Also location of the artifact in the time domain shifts slightly between channels (see Figure 7 and Figure 10).

In such a situation, the assumption is that mechanism that would diminish the results of the artifact in the outputs of the ResNet layers should improve the classification results as well as the network training process.

As can be seen in Figure 7 - Figure 10, ResNet layer output at different channels may differ. Because of this, attention acting in the time domain, i.e., detecting and mitigating results of the input artifact, must be fine-tuned for each channel separately.

Having output of the ResNet layer as tensor with shape C×F×T corresponding to (Channels, Frequency, Time), each time slice can be treated by the attention process separately. Having time slice with shape C×F×1 a dedicated neural network returns a vector of size C×1 that after being expanded to C×F×1 shape is positionally multiplied with the input time slice.

In this way, each channel across all frequencies is multiplied by the calculated channel-specific attention value for each time slice. Use of sigmoid activation function guarantees that attention value stays in (0,1) range.

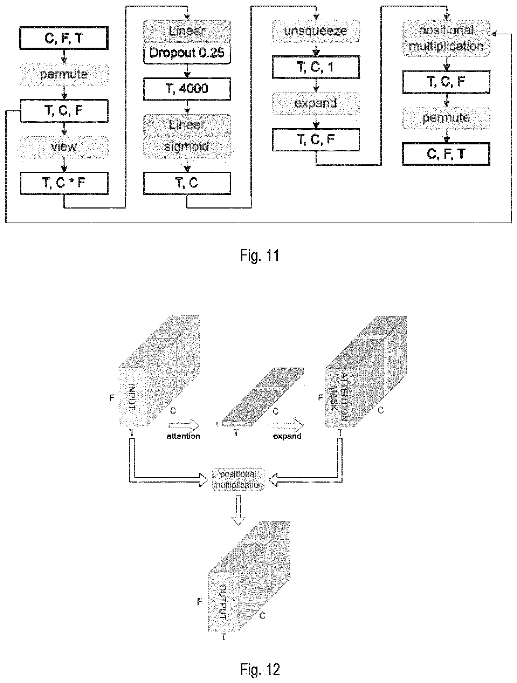

In PyTorch implementation, all time slices are, of course processed in parallel, as it is shown in Figure 11.

### Experiments

Network has been trained using real medical data obtained during 46 surgeries. During these surgeries 4650 recordings sampled with 24 kHz were registered. 3210 (69%) were labelled as recorded outside of the STN, while 1440 (31%) were labelled as recorded within the STN.

Training of the neural network has been done using Tesla v100 GPU using Python 3.8 with CUDA version 10.1, PyTorch library 1.8.1 and Ignite library 0.4.1.

Recordings were divided in a stratified way into disjoint training, validation, and test sets in proportion 8:1:1 accordingly. 80% of STN recordings and 80% of non-STN recordings were put into the training set. Half of the remaining STN recordings and half of the remaining non-STN recordings were put into the validation set. All remaining STN and non-STN recordings were put into the test set.

The recordings in each of the sets were then divided into overlapping chunks. Chunks of the length 12 000 samples (i.e. 500 ms) were taken with step 2 400 samples (i.e. 100 ms). In this way, a recording lasting 10 s yielded 96 chunks, each with a length of 0.5 s. The division into overlapping chunks acts as an augmentation and reduces the volume of the data put through the neural network at a given time.

After the augmentation process, a set of 412 348 chunks was obtained; there were 330 348 training chunks, 41 040 validation chunks, and 40 960 test chunks. As explained in scientific literature the most informative data for the localization of the STN resides in the frequency domain. For this reason, each chunk has been transformed into its spectrogram using the spectrogram method from the Python package SciPy. The window used for generating spectrograms was Tukey with shape parameter 0.25 and length 256. As a result, each chunk produces a spectrogram with dimensions 129 (frequency) by 53 (time), see Figure 2.

During the training, the cyclic learning rate has been applied with boundaries between 1e-8 and 1e-7. Cycle length has been set to match the length of an epoch. The stop condition for training was set to trigger after 25 consecutive epochs without a decrease of the loss value calculated for validation data.

The attention mechanism can also be visualized as calculation of T×C×1 attention tensor for input tensor of shape T×C×F. The attention tensor is then expanded to T×C×F shape and positionally multiplied with input to obtain the output tensor (see Figure 12).

One can notice on Figure 12 that for selected channel slice, there is an calculated attention vector of size T. This vector is then positionally multiplied across all frequencies of the selected channel slice. As the attention uses the sigmoid activation function, values of this vector are in the (0,1) range.

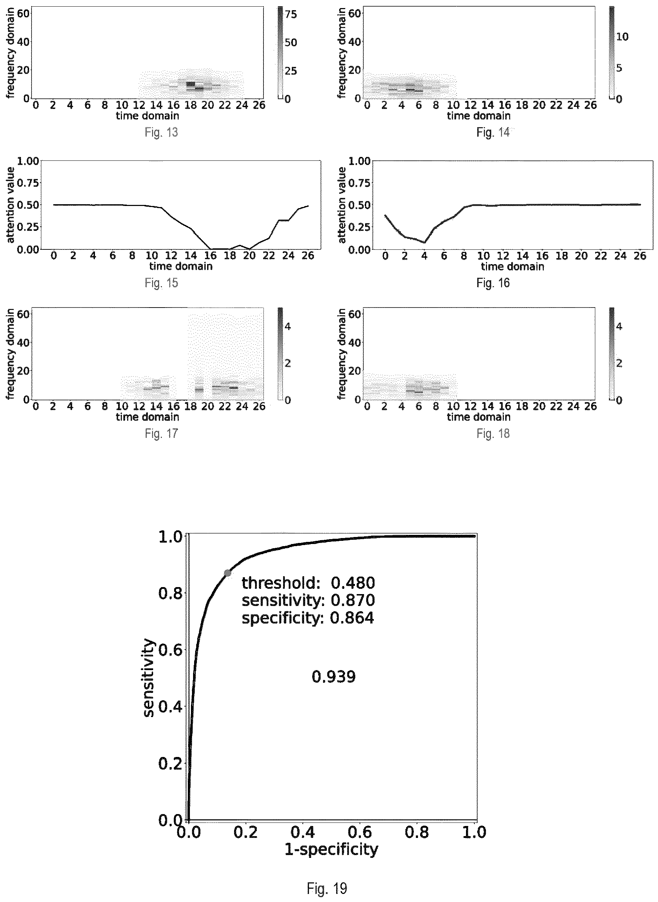

Figure 15 and Figure 16 show the values of the attention vector calculated by the trained network for selected ResNet layer outputs. One can readily see that for time ranges where the artifact is present, the value in the attention vector drops to mitigate it. It is also evident that for artifact with high amplitude (see Figure 13, time index 16-20) the value of the attention vector (see Figure 15, time index 16-20) is much smaller than in case of moderate artifact (see Figure 14 and Figure 16). s Figure 13 and Figure 14 show data that can be visualized as highlighted slice in INPUT in Figure 12. Figure 17 and Figure 18 show data that can be visualized as highlighted slice in OUTPUT in Figure 12.

Experiments shown that constructed attention mechanism is working. The attention mechanism emerges in an unsupervised way during the training, and it can work both for the diminishing of the artifacts and as a booster for other parts of the data passing through the neural network.

Finally, trained network was used for classification of the recordings from the training set of test set of 40 960 test chunks. The obtained AUC is 0.939 and the red point closest to upper-left corner (Figure 19) denotes sensitivity 0.870 and specificity 0.864. The classification threshold for the red point 0.48.

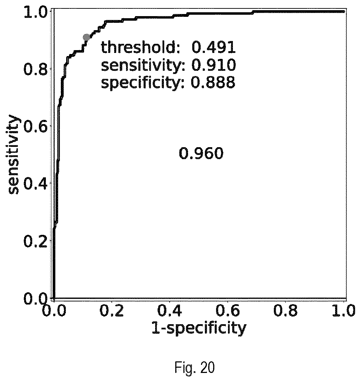

Classification of recordings is based upon the classification of the chunks obtained from them. The classification value for recording is a mean of results returned by the network for its chunks. The AUC for recordings (see Figure 20) is 0.96. The red point closest to the upper-left corner denotes the sensitivity of 0.91 and specificity of 0.888. The classification threshold for the above results is 0.491.

Classifier based upon described architecture achieved for chunks the AUC value above 0.9 which according to scientific literature is considered outstanding. In case of classification of the recording the AUC value is even higher i.e., above 0.95.

## Claims

1. A method of creating a computer implemented classification tool for classification of microelectrode recordings taken during a deep brain stimulation using deep residual neural network with attention comprising steps
collecting a data set of recordings taken during a deep brain stimulation;
splitting recordings into overlapping time chunks, and converting time chunks into spectrograms;
dividing data set into a training set, a validation set, and a test set putting each spectrogram into a deep neural network of ResNet architecture augmented with a self-attention layer added after each of ResNet layers, with a head layer comprising a single 2D convolutional layer followed by batch normalization and ReLU activation function wherein
the network is trained to return zero for time chunks taken from recordings made outside of the STN region of a brain and to return one for time chunks taken form recordings made within the STN region of a brain,
fine tuning the network with the validation set,
cross checking the network with the test set.

2. The method as in claim 1 wherein collecting data set includes signal recordings of 10 seconds.

3. The method as in claim 1 or 2 wherein the recordings are split into overlapping 500 ms long time chunks.

4. The method as in any of claims 1 to 3 wherein each spectrogram has dimensions 129 (frequency) by 53 (time).

5. The method as in any of claims 1 to 4 wherein the 2D convolutional layer has a kernel size 7x7, padding 3 and 64 filters.

6. The method as in any of claims 1 to 5 wherein each ResNet layer consists of six blocks, one lead-in block and five consecutive proper blocks.

7. The method as in any of claims 1 to 6 wherein the convolutional layers in ResNet blocks has kernel size 3x3 and padding 1.

8. A computer implemented classification tool for classification of microelectrode recordings taken during a deep brain stimulation, comprising
an input block for receiving signals from microelectrodes in STN region of a brain;
a conversion block for converting signals from the time domain into spectrograms;
a computer implemented a deep residual neural network with attention created as in any of claims from 1 to 7 for receiving spectrograms
an output block presenting the result of classification done by the deep residual neural network with attention.

## Patentansprüche

1. Ein Verfahren zur Erstellung eines computerimplementierten Klassifizierungswerkzeugs zur Klassifizierung von Mikroelektrodenaufzeichnungen, die während einer tiefen Hirnstimulation unter Verwendung eines tiefen residualen neuronalen Netzwerks mit Aufmerksamkeit durchgeführt wurde und folgenden Schritte umfasst:
Sammlung eines Datensatzes von Aufzeichnungen, die während einer tiefen Gehirnstimulation vorgenommen wurden;
Aufteilung der Aufnahmen in überlappende Zeitabschnitte und Umwandlung der Zeitabschnitte in Spektrogramme;
Aufteilung des Datensatzes in einen Trainingssatz, einen Validierungssatz, und einen Testsatz
Einfügen jedes Spektrogramms in ein tiefes neuronales Netzwerk der ResNet-Architektur, das mit einer Selbstbeobachtungsschicht erweitert ist, die nach jeder der ResNet-Schichten hinzugefügt wird, mit einer Hauptschicht, die eine einzelne 2D-Faltungsschicht gefolgt von Batch-Normalisierung und ReLU-Aktivierungsfunktion umfasst, wobei
das Netzwerk so trainiert wird, dass es Null für Zeitabschnitte aus Aufnahmen außerhalb der STN-Region eines Gehirns liefert und Eins für Zeitabschnitte aus Aufnahmen innerhalb der STN-Region eines Gehirns liefert, dazu erfolgt eine Feinabstimmung des Netzwerks mit dem Validierungsset, Kreuzung Überprüfung des Netzwerks mit dem Testsatz.

2. Das Verfahren wie in Anspruch 1 wobei das Sammeln von Daten Sätzen Signalaufzeichnungen von 10 Sekunden umfasst.

3. Das Verfahren wie in Anspruch 1 oder 2, wobei die Aufzeichnungen in überlappende, 500 ms lange, Zeitabschnitte gesplittet werden.

4. Das Verfahren wie in einem der Ansprüche 1 bis 3 wobei jedes Spektrogramm die Dimensionen 129 (Frequenz) mal 53 (Zeit) hat.

5. Das Verfahren wie in einem der Ansprüche 1 bis 4, wobei die 2D-Faltungsschicht eine Kernelgröße von 7×7, Padding 3 und 64 Filter hat.

6. Das Verfahren wie in einem der Ansprüche 1 bis 5, wobei jede ResNet Schicht aus sechs Blöcken, einem Lead-in Block und fünf aufeinanderfolgenden proper Blöcken besteht.

7. Das Verfahren wie in einem der Ansprüche 1 bis 6, wobei die Faltungsschichten in ResNet-Blöcken einen Kernel der Größe 3×3 und ein Padding 1 haben.

8. Ein computerimplementiertes Klassifizierungswerkzeug zur Klassifizierung von Mikroelektrodenaufzeichnungen, die während einer tiefen Hirnstimulation aufgenommen wurden, und folgendes umfassen:
einen Eingangsblock zum Empfang von Signalen von Mikroelektroden in der STN-Region eines Gehirns;
einen Umwandlungsblock zur Umwandlung von Signalen aus dem Zeitbereich in Spektrogramme;
ein computerimplementiertes ein tiefes residuales neuronales Netzwerk mit Aufmerksamkeit wie in einem der Ansprüche aus 1 bis 7 zum Empfang von Spektrogrammen
einen Ausgabeblock, der das Ergebnis der Klassifizierung darstellt, die durch das tiefe residuale neuronale Netzwerk mit Aufmerksamkeit durchgeführt wurde.

## Revendications

1. Une méthode de création d'outil de classification mis en oeuvre par ordinateur pour la classification des enregistrements par microélectrodes effectués au cours d'une stimulation cérébrale profonde utilisant le réseau neuronal résiduel profond avec attention comprenant les étapes suivantes :
la collecte d'un jeu de données d'enregistrements effectués lors d'une stimulation cérébrale profonde ;
la division des enregistrements en tranches de temps superposées et conversion les tranches de temps en spectrogrammes ;
la division du jeu de données en un ensemble d'apprentissage, un ensemble de validation et un ensemble test ;
le dépôt de chaque spectrogramme dans un réseau neuronal profond d'architecture ResNet augmenté d'une couche d'auto-attention ajoutée après chacune des couches de ResNet, avec une couche supérieure comprenant une couche simple 2D convolutive suivie d'une normalisation par lots et une fonction d'activation ReLU dans laquelle le réseau est entraîné à renvoyer zéro pour les morceaux de temps provenant d'enregistrements effectués en dehors de la région STN d'un cerveau et à renvoyer un pour les morceaux de temps provenant d'enregistrements effectués à l'intérieur de la région STN d'un cerveau, en ajustant finement le réseau avec l'ensemble de validation, en recoupant le réseau avec l'ensemble test.

2. La méthode selon la revendication 1 dans laquelle la collecte de données comprend des enregistrements de signaux de 10 secondes.

3. La méthode selon la revendication 1 ou 2 dans laquelle les enregistrements sont divisés en tranches de temps de 500 ms qui se chevauchent.

4. La méthode décrite dans l'une des revendications 1 à 3, dans laquelle chaque spectrogramme a des dimensions de 129 (fréquence) sur 53 (temps).

5. Le méthode décrite dans l'une des revendications 1 à 4, dans laquelle la couche convolutive 2D a un noyau de taille 7x7, un remplissage de 3 et 64 filtres.

6. La méthode décrite dans l'une des revendications 1 à 5, dans laquelle chaque couche ResNet se compose de six blocs, d'un bloc de début et de cinq blocs propres consécutifs.

7. La méthode comme dans l'une quelconque des revendications 1 à 6 dans laquelle les couches convolutives dans les blocs ResNet ont un noyau de taille 3x3 et un remplissage de 1.

8. Un outil de classification mis en oeuvre par ordinateur pour la classification des enregistrements par microélectrodes effectués au cours d'une stimulation cérébrale profonde, comprenant :
un bloc d'entrée pour la réception de signaux provenant de microélectrodes dans la région STN d'un cerveau ;
un bloc de conversion pour convertir les signaux du domaine temporel en spectrogrammes ;
un ordinateur mettant en oeuvre un réseau neuronal résiduel profond avec attention créé selon l'une des revendications 1 à 7 pour recevoir des spectrogrammes ;
un bloc de sortie présentant le résultat de la classification effectuée par le réseau neuronal résiduel profond avec attention.
